# FASCICULE DE BREVET EUROPEEN

(11) **EP 1 754 207 B1**
(45) Date de publication et mention de la délivrance du brevet: **12.08.2009**
(21) Numéro de dépôt: 05775269.3
(22) Date de dépôt: 02.06.2005
(51) Int. Cl.: G08C 17/02, A61B 5/22, A63B 24/00

(54) **DISPOSITIF DE TRAITEMENT D'INFORMATIONS ISSUES DE CAPTEURS ET/OU DESTINEES A DES ACTIONNEURS FAISANT INTERVENIR UN RADIOTELEPHONE MOBILE**
VORRICHTUNG ZUR VERARBEITUNG VON INFORMATIONEN AUS SENSOREN UND/ODER FÜR AKTUATOREN ZUR BETÄTIGUNG EINES MOBILFUNKTELEFONS
DEVICE FOR PROCESSING INFORMATION FROM SENSORS AND/OR INTENDED FOR ACTUATORS TRIGGERING A MOBILE RADIO TELEPHONE

(30) Priorité: 08.06.2004 FR 0406217
(43) Date de publication de la demande: 21.02.2007
(73) Titulaire: Jarry, Luc, F-78960 Voisins Le Bretonneux (FR); Prusker, Francis, 78590 Noisy Le Roi (FR)
(72) Inventeur: Jarry, Luc, F-78960 Voisins Le Bretonneux (FR); Prusker, Francis, 78590 Noisy Le Roi (FR)
(74) Mandataire: de Saint-Palais, Arnaud Marie
(86) Numéro de dépôt international: PCT/FR2005/001362
(87) Numéro de publication internationale: WO 2006/003279

(56) Documents cités:
- WO-A-01/41408
- WO-A-02/09404
- US-A1- 2004 002 634
- US-B1- 6 171 218

## Description

La présente invention concerne un dispositif pour le traitement d'informations issues de capteurs et/ou destinées à des actionneurs, faisant intervenir un radiotéléphone mobile.

Elle s'applique notamment mais non exclusivement à la conduite d'exercices sportifs basée sur l'utilisation de capteurs mesurant l'activité sportive d'un groupe d'utilisateurs et/ou d'actionneurs associés à l'équipement sportif exploité par l'utilisateur dudit groupe d'utilisateurs.

La conduite d'exercices sportifs faisant intervenir des capteurs mesurant l'activité sportive de l'utilisateur a été abordée de diverses manières.

Une première approche consiste à concevoir un système dédié comportant une pluralité de capteurs associés à une unité centrale embarquée, laquelle unité centrale traite les informations issues desdits capteurs implantés sur le corps de l'utilisateur et/ou sur l'équipement sportif exploité par l'utilisateur. La transmission des informations issues des capteurs s'effectue soit sur un support filaire soit sur un support hertzien dédié. La sélection des fonctionnalités de traitement et d'affichage des informations issues desdits capteurs est également dédiée. Quant à la transmission desdites données traitées vers une unité centrale distante et la restitution desdites données sur ladite unité centrale distante sont effectuées selon une procédure également dédiée.
Cette approche, largement exploitée dans le domaine du sport individuel, ne permet pas d'adapter la capture et le traitement d'informations en fonction de la nature des efforts physiques effectués, de l'évolution des capacités physiques de l'utilisateur, ou tout simplement du type de sport pratiqué.

Une seconde approche, de préférence statique, consiste à effectuer une biométrie sur l'utilisateur, ou plus précisément sur le patient ; cette approche est essentiellement exploitée dans le domaine médical ; les systèmes exploités sont dédiés et adaptés aux modes d'analyse et d'interprétation propres aux experts du domaine concerné selon des critères préétablis et reconnus.
Cette approche ne permet pas une relation bidirectionnelle autorisant notamment de déclencher, par contre-réaction automatique ou délibérée, des actionneurs permettant de modifier les conditions de tests.

Une troisième approche consiste à exploiter des équipements mobiles sophistiqués, tels des PDA (Personal Digital Assistant) ou des radiotéléphones mobiles haut de gamme (Smartphone) disposant d'interfaces permettant d'ajouter des périphériques.
Les spécifications de ces interfaces sont normalisées et définissent les caractéristiques des périphériques associés auxdits équipements mobiles. A titre d'exemple, il existe à ce jour des accessoires permettant de se localiser (GPS), de transmettre en mode bidirectionnel, sur courte distance, la voix sur support hertzien (Bluetooth), etc.
Cette approche est également de nature dédiée ; elle n'est en aucune manière évolutive et n'autorise pas l'extension à d'autres périphériques.

L'invention a pour but d'éviter une démarche essentiellement dédiée, monodirectionnelle et accessible uniquement à des experts ; elle propose à cet effet une démarche évolutive, configurable par l'utilisateur, associée à des capteurs et/ou des actionneurs, utilisant un radiotéléphone mobile et notamment son interface homme/machine caractérisée par ses capacités de traitement, de stockage, d'affichage et de transmission bidirectionnelle.

Le document WO 02/09404 A1 dévoile un dispositif de réception et de transmission de données et de transmission de commandes, constitué d'un appareil et d'un téléphone portable, en vue de commander, déclencher ou mettre en ouvre des fonctions d'actionneurs ou de recevoir des informations de capteurs distants connectés audit appareil. Une particularité de ce dispositif est que l'appareil, qui remplit la fonction d'interface entre les capteurs et actionneurs d'une part et un téléphone portable d'autre part, peut être appelé par plusieurs utilisateurs avec des téléphones portables différents, et être configuré pour accorder des droits différents selon le numéro appelant. Les capteurs et actionneurs sont invariables ; une fois l'appareil, les capteurs et les actionneurs installés, l'utilisateur n'en change plus. Les téléphones portables utilisés sont standards et ne comportent pas de moyens de configuration de l'appareil.
Le document US 2004/0002634 A1 dévoile un système comprenant un ou plusieurs senseur(s) physiologique(s) captant des données concernant un utilisateur, par exemple son poids, sa consommation calorique ou sa pression sanguine, ainsi qu'un dispositif d'émission couplé audit senseur communiquant avec un téléphone portable, lequel peut communiquer avec un serveur d'informations qui peut traiter les données physiologiques reçues du ou des senseur(s) et les intégrer dans un modèle physiologique de l'utilisateur ou avatar. En retour, l'utilisateur reçoit du serveur, sur son téléphone portable, des informations concernant sa forme physique.
Le document WO 01141408 A1 dévoile un appareil et un procédé pour commander un appareil électrique à partir d'un téléphone portable. Ce document fait intervenir une interface Module Serveur WAP connectée d'une part à un appareil électrique assimilable à un actionneur, d'autre part à un téléphone portable. Le Module Serveur WAP est standard et configurable en fonction de l'appareil électrique auquel il est connecté, par le biais de pages WML qui sont stockées dans l'appareil électrique et qui lui sont transférées. Il ressort clairement de ce document que le téléphone portable n'est que client WAP, son utilisateur ne peut envoyer que des commandes aux appareils électriques.
Selon l'invention, ce dispositif pour le traitement d'informations issues de capteurs et/ou destinées à des actionneurs faisant intervenir un radiotéléphone mobile est défini dans les revendications 1-8.

Un mode d'exécution de l'invention sera décrit ci-après, à titre non limitatif, avec référence aux dessins annexés, dans lesquels :
- la figure 1 est une représentation schématique du dispositif selon l'invention,
- la figure 2 est une représentation schématique de l'architecture matérielle du dispositif selon l'invention.

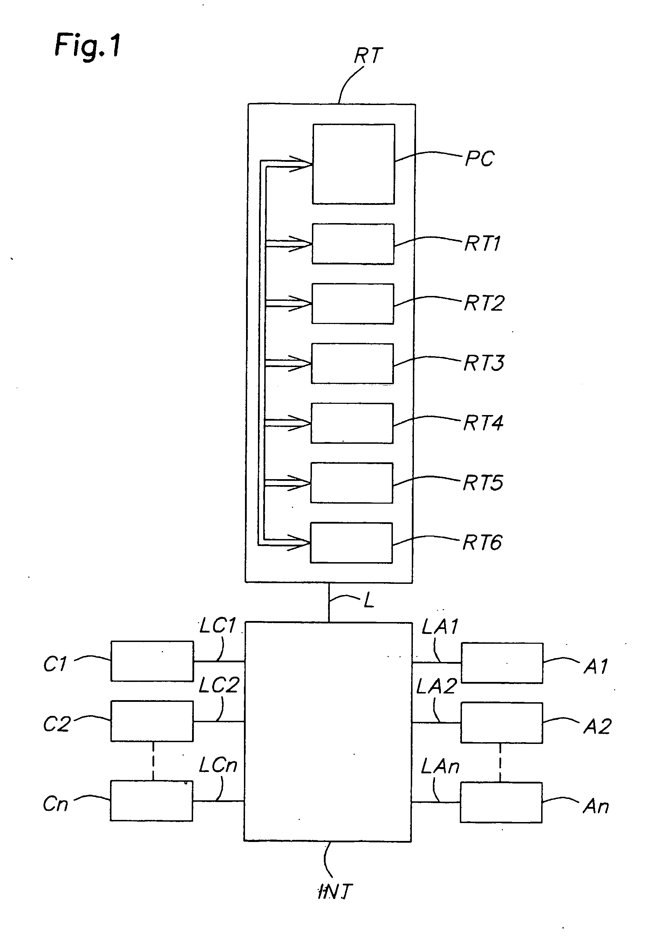

Dans l'exemple représenté sur la figure 1, une pluralité de capteurs (C1, C2,...Cn) et une pluralité d'actionneurs (A1, A2,...An) sont associés à des moyens d'interfaçage INT, par l'intermédiaire, respectivement de moyens de liaisons LC1, LC2,...LCn et LA1, LA2,...LAn.
En outre, un radiotéléphone mobile RT, comprenant :
- des moyens de configuration RT1 desdits moyens d'interfaçage INT,
- des moyens de téléchargement RT2 d'un programme de traitement des informations issues desdits moyens d'interfaçage INT, et/ou transmises auxdits moyens d' interfaçage INT,
- des moyens de stockage RT3 desdites informations issues desdits moyens d' interfaçage INT, et/ou transmises auxdits moyens d'interfaçage INT,
- des moyens de traitement RT4 desdites informations issues desdits moyens d'interfaçage INT, et/ou transmises auxdits moyens d' interfaçage INT,
- des moyens d'affichage RT5 desdites informations issues desdits moyens d'interfaçage INT, et/ou transmises auxdits moyens d' interfaçage INT, et
- des moyens d'émission RT6 des informations issues desdits moyens d'interfaçage INT, et/ou de réception des informations transmises auxdits moyens d'interfaçage,
est associé aux moyens d'interfaçage INT par l'intermédiaire de moyens de liaison L.

Les susdits moyens d'interfaçage INT permettent d'associer deux domaines techniques de nature très différente : le domaine des radiotéléphones mobiles et le domaine des capteurs et actionneurs ; ces deux domaines ont leur dynamique propre et obéissent à des règles différentes tant au niveau des interfaces utilisées que concernant les modes de connexion.
Dans le sens de la "voie montante", c'est-à-dire dans le sens capteur vers radiotéléphone mobile, les moyens d'interfaçage INT sont chargés de centraliser les données des capteurs, de les traiter et de les communiquer au radiotéléphone mobile RT par l'intermédiaire des moyens de liaison L. Ainsi les données brutes issues des capteurs sont transformées en des valeurs significatives et accessibles à la compréhension dans le domaine concerné. A titre d'exemple, une séquence d'impulsions de périodicité constante ou variable, peut être interprétée comme un battement de coeur, une cadence de pédalage, une vitesse de rotation d'une roue, etc.
Dans le sens de la "voie descendante", c'est-à-dire dans le sens radiotéléphone mobile vers actionneur, les moyens d'interfaçage INT reçoivent les informations émanant du radiotéléphone mobile RT par l'intermédiaire des moyens de liaison L, et les transforment en des données de commande compatibles avec les actionneurs concernés. A titre d'exemple, au cours d'un exercice de pédalage sur un vélo, la commande peut concerner le changement de vitesse du pédalier jugé nécessaire suite à l'analyse du rythme cardiaque.

Le susdit radiotéléphone mobile RT comprend les moyens RT1, RT2, RT3, RT4, RT5, RT6, cités précédemment ; ces différents moyens peuvent être de nature purement matérielle, ou de nature purement logicielle, ou de nature matérielle et logicielle. Ces susdits moyens sont connus en soi ; ils sont gérés par un processeur central PC par l'intermédiaire d'un bus de liaison interne.
Par ailleurs, l'interface homme/machine permettant d'exploiter les capacités de traitement, de stockage et de transmission bidirectionnelle, est constituée essentiellement d'un écran d'affichage de données numériques et d'un clavier.

Avantageusement, le radiotéléphone mobile RT sera en relation, par l'intermédiaire d'un réseau cellulaire, avec un terminal de type téléphonique, ou de type unité centrale, ou de type serveur, etc.
Ainsi, les informations émises par le radiotéléphone RT pourront être traitées soit en temps réel, soit ultérieurement par un expert, ou pourront être traitées d'une manière automatique par une unité de traitement de type serveur.
Réciproquement, les informations reçues par le radiotéléphone tel un programme de traitement à télécharger, pourront être émises par un serveur ; de même, les informations reçues par le radiotéléphone tel des ordres de commande d'actionneurs, pourront être émises par un expert en temps réel.

Les susdites liaisons LC1, LC2,...LCn, d'une part LA1, LA2,...LAn d'autre part, pourront être de type liaison filaire, de type liaison radio, de type liaison optique ; elles pourront effectuer des transmissions de données pour un capteur individuel ou un actionneur individuel, ou effectuer des transmissions de données pour un groupe de capteurs ou un groupe d'actionneurs, lesquelles données seront multiplexées puis démultiplexées pour lesdits groupes de capteurs et d'actionneurs.

La susdite liaison L pourra être de type liaison filaire aux normes "RS232" ou autres, de type liaison optique infrarouge, de type de liaison radio aux normes "Bluetooth", "Wi-Fi", ou autres.

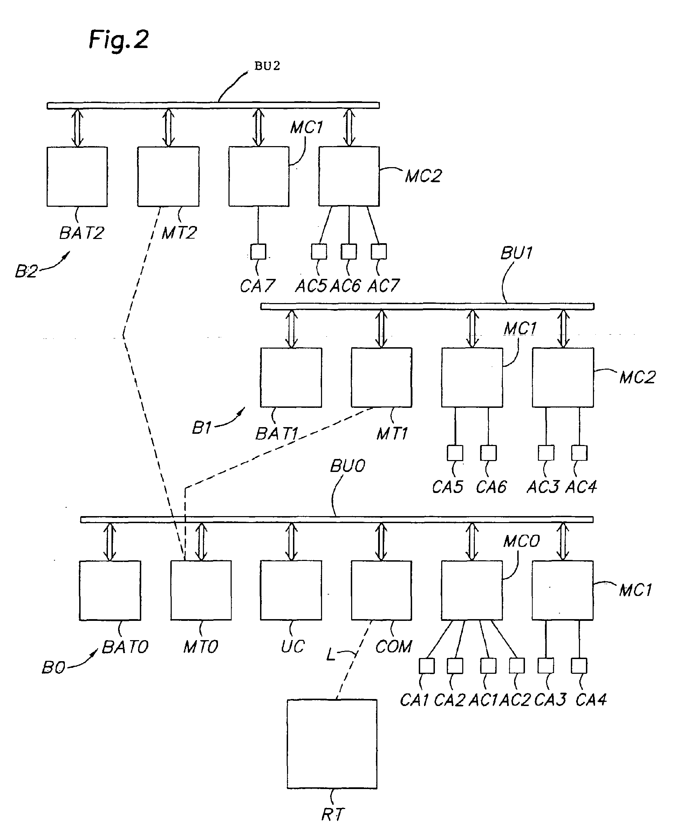

Dans l'exemple représenté sur la figure 2, trois blocs B0, B1, B2, constituent, à titre d'exemple, une architecture matérielle du dispositif selon l'invention.

Le bloc B0, dit principal, comprend une pluralité de modules BAT0, MT0, UC, COM, MC0, MC1, lesquels modules sont solidaires d'un bus BU0 de liaison de données et d'alimentation.

Cet ensemble constitue un bloc physique autonome capable de traiter des informations issues de capteurs et/ou destinées à des actionneurs, lequel ensemble est associé à un radiotéléphone mobile RT.

Les deux autres blocs B1, B2, dits secondaires, comprennent également une pluralité de modules, respectivement BAT1, MT1, MC1, MC2, et BAT2, MT2, MC1, MC2, et constituent à leur tour des blocs physiques autonomes distants du susdit bloc principal B0, en relation bidirectionnelle avec le susdit bloc B0 au moyen de liaisons radiofréquence, et capables de traiter des informations issues de capteurs et/ou destinées à des actionneurs.
Les susdits blocs secondaires B1, B2, comprennent également des bus de liaison de données et d'alimentation, respectivement BU1, BU2, associant leurs propres modules précédemment cités.

Les susdits modules MC0, MC1, MC2, constituent l'interfaçage entre le bus de liaison de données et les capteurs et/ou les actionneurs ; d'une manière plus précise, ils comprennent :
- l'interface bus,
- l'interface capteurs et/ou actionneurs,
- le multiplexage des informations issues des capteurs, et/ou
- le démultiplexage des informations transmises aux actionneurs,
- une unité centrale de type microcontrôleur.

Ainsi, le module MC0 comprend l'ensemble des fonctionnalités définies ci-dessus, le module MC 1 est associé uniquement à des capteurs, le module MC2 est associé uniquement à des actionneurs.

Le module MC 1 comprend :
- l'interface bus,
- l'interface capteurs,
- le multiplexage des informations issues des capteurs,
- une unité centrale de type microcontrôleur.
Le module MC2 comprend :
- l'interface bus,
- l'interface actionneurs,
- le démultiplexage des informations transmises aux actionneurs,
- une unité centrale de type microcontrôleur.

Dans l'exemple représenté sur la figure 2, le module MC0 du bloc principal B0 est associé aux capteurs CA1, CA2, et aux actionneurs AC1, AC2.
Le module MC 1 du bloc principal B0 est associé aux capteurs CA3, CA4.
Les modules MC1, MC2, du bloc secondaire B1 sont associés respectivement aux capteurs CA5 CA6 et aux actionneurs AC3, AC4.
Les modules MC1 MC2, du bloc secondaire B2 sont associés respectivement au capteur CA7 et aux actionneurs CA5 AC6, AC7.

Les modules MT0, MT1, MT2, permettent la liaison radiofréquence entre les différents blocs B0, B1, B2 ; d'une manière plus précise, ils comprennent :
- l'interface bus,
- un émetteur/récepteur radiofréquence.
Ainsi le module MT0 est en liaison radiofréquence avec lesdits modules MT1, MT2, permettant au bloc principal B0 de recevoir les informations issues des capteurs associés aux susdits blocs secondaires B1, B2, et de transmettre aux mêmes blocs les informations destinées à leurs actionneurs.

Le module COM du bloc principal B0 constitue l'interfaçage dudit bloc principal B0 et du radiotéléphone RT par l'intermédiaire de la liaison L bidirectionnelle, filaire, ou infrarouge ou radiofréquence.

Le module UC du bloc principal B0 constitue l'unité centrale dudit bloc principal B0 et comprend au moins les fonctionnalités suivantes :
- la gestion du bus BU0,
- l'identification des capteurs CA1, CA2, CA3, CA4, et des actionneurs AC1, AC2,
- la récupération du programme de traitement des informations issues des capteurs et celles transmises aux actionneurs,
- le traitement des informations issues des capteurs CA1, CA2, CA3, CA4 et celles transmises aux actionneurs AC1, AC2,
- le traitement des informations issues des capteurs associés aux blocs secondaires B1, B2, et des informations transmises aux actionneurs associés aux blocs secondaires B1, B2,
- la transmission bidirectionnelle des susdites informations, par l'intermédiaire du bus BU0 au module COM.

Les modules BAT0, BAT1, BAT2, permettent l'alimentation électrique des différents modules associés, au travers du bus correspondant.

Avantageusement, ces différents modules pourront être de dimensions identiques ou quasi-identiques, tels des parallélépipèdes de faibles dimensions (20 mm de côté, 3 mm d'épaisseur, à titre d'exemple) et comportant une connectique identique permettant la liaison avec le bus correspondant.
Le bus, sous son aspect physique, pourra être semblable à une bande de faibles dimensions transversales, telle une ceinture et comportant un nombre suffisant de ports ; le protocole de liaison sera identique pour la totalité des bus utilisés, permettant ainsi de positionner indifféremment les capteurs et de modifier le nombre de capteurs sur chacun des bus en fonction de l'exploitation recherchée desdits capteurs et/ou desdits actionneurs.

Ainsi, le dimensionnement quasi identique des modules, la connectique desdits modules sur le bus, le protocole de liaison entre les différents modules exploité par les bus de liaison, l'interconnexion radiofréquence entre les différents blocs, permettent :
- la constitution de blocs adaptés au nombre de capteurs et/ou d'actionneurs à exploiter,
- l'association de plusieurs blocs secondaires tels B1 B2, au bloc principal B0
- l'ajout de nouveaux blocs secondaires.

Les susdits modules MC0, MC1, MC2, sont destinés à l'interfaçage des capteurs et/ou des actionneurs avec le bus correspondant.
En effet, les capteurs et les actionneurs peuvent être de type analogique ou numérique.

D'une manière plus précise, un capteur, en fonction de la grandeur physique mesurée, peut délivrée une information analogique proportionnelle à la grandeur mesurée, ou une information numérique représentant la grandeur mesurée sous forme d'un nombre booléen, ou une information de type périodique sous forme d'un train d'impulsions.
De même un actionneur peut agir à partir d'une information analogique, ou à partir d'une information numérique, ou en mode "tout ou rien".
A titre d'exemple, un capteur de type biométrique, délivre des impulsions correspondant au rythme cardiaque, à la contraction musculaire, au clignement de l'oeil, etc.
Un capteur analogique délivre une tension proportionnelle à la pression atmosphérique absolue ou relative, à la vitesse du vent, à la température du corps, etc.

Un actionneur, fonctionnant en mode "tout ou rien", peut être le changeur de vitesse d'un pédalier de vélo, une alarme sonore, etc.

Ainsi, les informations issues des capteurs ou transmises aux actionneurs peuvent prendre plusieurs significations selon la nature des capteurs ou des actionneurs considérés.
Il est donc nécessaire de définir l'interprétation des informations issues des capteurs ou transmises aux actionneurs, préalablement à l'exploitation desdits capteurs et actionneurs.
L'utilisateur devra donc effectuer une configuration des différents capteurs et actionneurs préalablement à la première utilisation du dispositif ; en effet un capteur de type accéléromètre peut mesurer, par exemple, le nombre de foulées par minute du coureur ou la vitesse de rotation d'un pédalier de vélo ; la transformation de l'information issue de l'accéléromètre sera différente, s'agissant du nombre de foulées ou de la vitesse de rotation du pédalier ; l'interprétation des résultats sera différente, concernant par exemple la quantification de l'énergie consommée par le sportif, laquelle est une fonction linéaire du nombre de foulées et une fonction discontinue de la vitesse de rotation du pédalier, étant donné que ladite vitesse de rotation du pédalier est définie par le rapport du changeur de vitesse du vélo, lequel rapport est déterminé par la pente gravie par le cycliste.
A ce titre, des moyens de configuration RT1 sont prévus à cet effet dans le radiotéléphone mobile RT, associés à l'interface homme/machine constituée d'un écran d'affichage de données numériques et d'un clavier.
Avantageusement une description de la configuration sera effectuée en attribuant à chacun des capteurs et actionneurs constituants le dispositif, une fonction réelle à partir d'un choix proposé, le domaine de fonctionnement et autres paramètres utiles, et une identification par un code ou un en-tête attribué.
Cette configuration est bien évidemment compatible avec les capteurs et les actionneurs choisis.

Le programme de traitement pourra être disponible au niveau de l'unité centrale du radiotéléphone mobile RT, ou pourra être téléchargé par ledit radiotéléphone mobile RT grâce aux susdits moyens de téléchargement RT2, à partir d'un serveur, d'un terminal équipé d'une unité centrale d'un expert, etc. Il effectuera le stockage des informations reçues et transmises grâce aux susdits moyens de stockage RT3, puis en temps réel ou différé, le traitement des informations reçues ou transmises grâce aux susdits moyens de traitement RT4.
Lesdites informations reçues ou transmises ainsi que les traitements associés seront affichés grâce aux susdits moyens d'affichage RT5 et enfin transmises ou reçues grâce aux susdits moyens d'émission/réception RT6.

Ainsi, à la possibilité de constituer plusieurs blocs secondaires adaptés au nombre de capteurs et/ou d'actionneurs et de les associer au bloc principal, s'ajoute la possibilité de destiner l'ensemble desdits blocs principal et secondaires à plusieurs types d'exercice sportif après configuration du susdit ensemble et chargement d'un programme de test adapté à l'exercice physique souhaité. L'exploitation des résultats pourra être effectuée en temps réel, in situ ou transférée vers une unité centrale ou un serveur dédié. Une adaptation du susdit ensemble sera toujours possible suite à l'exploitation desdits résultats.

Par ailleurs, les liaisons radiofréquence entre les modules MT0 et MT1 d'une part, et entre les modules MT0 et MT2 d'autre part, permettant au bloc principal B0 de recevoir les informations issues des capteurs associés aux susdits blocs secondaires B1, B2, et de transmettre aux mêmes blocs les informations destinées à leurs actionneurs, peuvent être suffisamment distantes de manière à ce que les susdits blocs secondaires B 1, B2, soient destinés à des utilisateurs différents, distants de l'utilisateur équipé du bloc B0

Ainsi, dans le cadre d'une activité sportive par équipe, les membres de l'équipe pourront être gérés en temps réel par le responsable de ladite équipe, lequel est équipé du bloc B0.

A titre d'exemple, durant une course à pied ou à vélo, le responsable de l'équipe de coureurs pourra entre autre localiser les différents membres de l'équipe à partir de la détermination des distances parcourues par chacun des membres ; lesquelles distances parcourues seront calculées à partir des données issues des capteurs des blocs correspondants aux différents membres de l'équipe.

Le dispositif, selon l'invention, constitue par conséquent un ensemble modulaire, adaptable à tout type de capteur et/ou d'actionneur, programmable en fonction de la nature des exercices à effectuer, exploitable in situ ou à distance, grâce à l'association d'un radiotéléphone mobile.

## Revendications

1. Système pour la transmission et le traitement d'informations, ce système comprenant un radiotéléphone mobile (RT) et des moyens d'interfaçage (INT) distants dudit radiotéléphone, lesdits moyens d'interfaçage étant reliés à au moins un capteur (C1, C2, ...Cn) et/ou au moins un actionneur (A1, A2, ...An) par des moyens de liaison (LC1, LC2...LCn, LA1, LA2...LAn), lesdits
moyens de liaison (L) permettant la transmission entre les susdits moyens d'interfaçage (INT) et le susdit radiotéléphone mobile (RT),
de données fournies par ledit au moins un capteur ou de données de commande destinées audit au moins un actionneur,
**caractérisé en ce que** le radiotéléphone (RT) comporte :
• une unité centrale (PC),
• des moyens de configuration (RT1) desdits moyens d'interfaçage (INT) en fonction du au moins un capteurs et/ou du au moins un actionneurs auxquels lesdits moyens d'interfaçage sont reliés et en fonction du domaine d'application dudit système,
• des moyens de téléchargement (RT2) au sein de ladite unité centrale (PC) d'un programme de traitement adapté des données issues desdits moyens d' interfaçage (INT) ou des données de commande transmises auxdits moyens d'interfaçage (INT),
• des moyens de stockage (RT3) desdites données dudit au moins un capteur issues desdits moyens d'interfaçage (INT), et/ou des données de commande transmises auxdits moyens d'interfaçage (INT),
• des moyens de traitement (RT4) desdites données issues desdits moyens d'interfaçage (INT), et/ou transmises auxdits moyens d' interfaçage (INT),
• des moyens d'affichage (RT5) desdites données issues desdits moyens d'interfaçage (INT), et/ou transmises auxdits moyens d'interfaçage (INT), et
• des moyens d'émission (RT6) des données issues desdits moyens d'interfaçage (INT) vers un réseau cellulaire et/ou de réception des données destinées auxdits moyens d'interfaçage (INT) et en provenance dudit réseau cellulaire,
**en ce que** les moyens d'interfaçage (INT) comprennent :
• au moins un module d'interface (MC0, MC1, MC2) avec ledit au moins un capteur et/ou au moins un actionneurs, et
• un module de communication (COM) pour la mise en oeuvre d'une liaison (L) avec ledit radiotéléphone portable (RT),
et **en ce que** ledit au moins un modules d'interface (MC0, MC1, MC2) desdits moyens d' interfaçage (INT) est configurable grâce auxdits moyens de configuration (RT1) du radiotéléphone portable (RT), les moyens de configuration (RT1) effectuant une description de la configuration visant à l'attribution à chacun des au moins un capteur et au moins un actionneur constituant le dispositif d'une fonction réelle à partir d'un choix proposé, d'un domaine de fonctionnement et d'une identification.

2. Système selon la revendication 1,
**caractérisé en ce que** ledit programme de traitement effectue le stockage des données de capteur et/ou des données de commande grâce auxdits moyens de stockage (RT3) du radiotéléphone (RT) et leur affichage grâce auxdits moyens d'affichage (RT5) du radiotéléphone (RT),

3. Système selon la revendication 2,
**caractérisé en ce que** ledit programme de traitement est adapté à la conduite d'exercices sportifs.

4. Système selon l'une quelconque des revendications précédentes,
**caractérisé en ce que** lesdits moyens d'interfaçage (INT) comprennent une pluralité de blocs d'interface (B0, B1, B2...), un bloc d'interface principal (B0) et au moins un bloc d'interface secondaire (B1, B2...), lesdits blocs d'interface comportant des moyens de liaison radiofréquence (MT0, MT1, MT2...) de façon à permettre l'échange de données entre le bloc d'interface principal (B0) d'une part et chacun des blocs d'interface secondaires d'autre part, le bloc principal (B0) servant de relais à la fois pour la transmission de données de capteurs fournies par les blocs d'interface secondaires (B1, B2...) à destination du radiotéléphone portable (RT) et pour la transmission de données de commande fournies par le radiotéléphone portable (RT) à destination des blocs d'interface secondaires (B1, B2...).

5. Système selon l'une des revendications précédentes,
**caractérisé en ce que** lesdits moyens de liaison (LC1, LC2,...LCn) entre ledit module d'interfaçage (INT) et ledit au moins un capteur, et/ou lesdits moyens de liaison (LA1, LA2,...LAn) entre ledit module d'interfaçage (INT) et ledit au moins un actionneur, sont de type liaison filaire, ou de type liaison radio, ou de type liaison optique.

6. Système selon l'une quelconque des revendications précédentes,
**caractérisé en ce que** lesdits moyens de liaison (L) entre ledit module d'interfaçage (INT) et ledit radiotéléphone mobile (RT), sont de type liaison filaire aux normes "RS232", ou de type liaison optique infrarouge, ou de type de liaison radio aux normes "Bluetooth", "Wi-Fi".

7. Système selon l'une quelconque des revendications précédentes,
**caractérisé en ce que** ledit module (COM) constitue l'interface dudit bloc (B0) et dudit radiotéléphone (RT).

8. Système selon l'une quelconque des revendications précédentes,
**caractérisé en ce qu'**il permet la conduite d'exercices sportifs, ledit au moins un capteur mesurant l'activité sportive d'un groupe d'utilisateurs, ledit au moins un actionneur étant associé aux équipements sportifs exploités par les membres dudit groupe d'utilisateurs.

## Claims

1. A system for the transmission and processing of information, this system comprising a mobile radiotelephone (RT) and interfacing means (INT) remote from said radiotelephone, said interfacing means being connected to at least one sensor (C1, C2, ... Cn) and/or to at least one actuator (A1, A2, ... An) by connecting means (LC1, LC2...LCn, LA1, LA2, ...LAn), said
connecting means (L) allowing the transmission between the aforementioned interfacing means (INT) and the aforementioned mobile radiotelephone (RT),
of data provided by said at least one sensor or control data intended for said at least one actuator,
**characterized in that** the radiotelephone (RT) comprises:
• a central unit (PC),
• means (RT1) for configuring said interfacing means (INT) as a function of said at least one sensor and/or said at least one actuator to which said interfacing means are connected and as a function of the field of application of the system,
• downloading means (RT2) within said central unit (PC) of a processing program adapted for the data coming from said interfacing means (INT) or for the control data transmitted to said interfacing means (INT),
• means (RT3) for storing said data from said at least one sensor coming from said interfacing means (INT), and/or control data transmitted to said interfacing means (INT),
• means (RT4) for processing said data coming from said interfacing means (INT), and/or transmitted to said interfacing means (INT),
• means (RT5) for displaying said data coming from said interfacing means (INT), and/or transmitted to said interfacing means (INT), and
• means (RT6) for transmitting data coming from said interfacing means (INT) to a cellular network and/or for receiving data bound for said interfacing means (INT) and coming from said cellular network,
**in that** the interfacing means (INT) comprise:
• at least one interface module (MC0, MC1, MC2) with said at least one sensor and/or at least one actuator, and
• a communication module (COM) for the implementation of a connection (L) with said portable radiotelephone (RT),
and **in that** said at least one interface module (MC0, MC1, MC2) of said interfacing means (INT) is configurable using said configuration means (RT1) of the portable radiotelephone (RT), the configuration means (RT1) performing a description of the configuration aiming to assign each at least one sensor and at least one actuator constituting the device a real function from a proposed choice, a field of operation and an identity.

2. The system according to claim 1,
**characterized in that** said processing program performs the storage of sensor data and/or control data using said storage means (RT3) of the radiotelephone (RT) and displays them using said display means (RT5) of the radiotelephone (RT).

3. The system according to claim 2,
**characterized in that** said processing program is adapted to the performance of athletic exercises.

4. The system according to any one of the preceding claims,
**characterized in that** said interfacing means (INT) comprise a plurality of interface blocks (B0, B1, B2, ...), a primary interface block (B0) and at least one secondary interface block (B1, B2...), said interface blocks comprising radiofrequency connecting means (MT0, MT1, MT2...) so as to enable the exchange of data between the primary interface block (B0) on one hand and each of the secondary interface blocks on the other hand, the primary block (B0) serving as a relay both for the transmission of sensor data supplied by the secondary interface blocks (B1, B2...) bound for the portable radiotelephone (RT) and for the transmission of control data supplied by the portable radiotelephone (RT) bound for the secondary interface blocks (B1, B2...).

5. The system according to one of the preceding claims,
**characterized in that** said connecting means (LC1, LC2, ... LCn) between sad interfacing module (INT) and said at least one sensor, and/or said connecting means (LA1, LA2, ... LAn) between said interfacing module (INT) and said at least one actuator, are of the fixed connection type, or the radio connection type, or the optical connection type.

6. The system according to any one of the preceding claims,
**characterized in that** said connecting means (L) between said interfacing module (INT) and said mobile radiotelephone (RT) are of the fixed connection type at the "RS232" standards, or of the infrared optical connection type, or of the radio connection type at the "Bluetooth", "Wi-Fi" standards.

7. The system according to any one of the preceding claims,
**characterized in that** said module (COM) constitutes the interface of said block (B0) and said radiotelephone (RT).

8. The system according to any one of the preceding claims,
**characterized in that** it allows the performance of athletic exercises, said at least one sensor measuring the athletic activity of a group of users, said at least one actuator being connected to the athletic equipment used by the members of said group of users.

## Patentansprüche

1. System zur Übertragung und Verarbeitung von Informationen, wobei dieses System ein Mobilfunktelefon (RT) und von dem drahtlosen Telefon entfernte Kopplungsmittel (INT), wobei die Kopplungsmittel mit mindestens einem Sensor (C1, C2,...Cn) und/oder mit mindestens einem Schalter (A1, A2,...An) über Verbindungsmittel (LC1, LC2...LCn, LA1, LA2...LAn) verbunden sind, wobei die Verbindungsmittel (L) die Übertragung zwischen den oben genannten Kopplungsmitteln (INT) und dem oben genannten Mobilfunktelefon (RT) ermöglichen,
Daten, die von dem mindestens einen Sensor bereitgestellt werden, oder Steuerungsdaten, die für den mindestens einen Schalter bestimmt sind, umfasst;
**dadurch gekennzeichnet, dass** das Funktelefon (RT) folgendes umfasst:
• eine Zentraleinheit (PC),
• Konfigurierungsmittel (RT1) für die Kopplungsmittel (INT) in Funktion des mindestens einen Sensors und/oder des mindestens einen Schalters, mit welchen die Kopplungsmittel verbunden sind, und in Funktion des Anwendungsbereichs des Systems,
• Fernlademittel (RT2) an der Zentraleinheit (PC) eines Verarbeitungsprogramms, das für die von den Kopplungsmitteln (INT) ausgegebenen Daten oder für die zu den Kopplungsmitteln (INT) übertragenen Steuerungsdaten ausgelegt ist,
• Speichermittel (RT3) für die Daten des mindestens einen Sensors, die von den Kopplungsmitteln (INT) ausgegeben werden, und/oder für die zu den Kopplungsmitteln (INT) übertragenen Steuerungsdaten,
• Verarbeitungsmittel (RT4) für die von den Kopplungsmitteln (INT) ausgegebenen und/oder für die zu den Kopplungsmitteln (INT) übertragenen Daten,
• Anzeigemittel (RT5) für die von den Kopplungsmitteln (INT) ausgegebenen Daten und/oder für die zu den Kopplungsmitteln (INT) übertragenen Daten, und
• Ausgabemittel (RT6) für die von den Kopplungsmitteln (INT) zu einem Mobilfunknetz ausgegebenen Daten und/oder Empfangsmittel für die Daten, die für die Kopplungsmittel (INT) bestimmt sind und die von dem Mobilfunknetz kommen,
**dadurch**, dass die Kopplungsmittel (INT) folgendes umfassen:
• mindestens ein Schnittstellen-Modul (MC0, MC1, MC2) mit dem mindestens einen Sensor und/oder dem mindestens einen Schalter, und
• ein Kommunikationsmodul (COM) für die Realisierung einer Verbindung (L) mit dem tragbaren Funktelefon (RT),
und **dadurch**, dass das mindestens eine Schnittstellen-Modul (MC0, MC1, MC2) der Kopplungsmittel (INT) mit Hilfe der Konfigurierungsmittel (RT1) des tragbaren Funktelefons (RT) konfigurierbar ist, wobei die Konfigurierungsmittel eine Beschreibung der Konfigurierung durchführen, die auf die Zuweisung, jeweils zu dem mindestens einen Sensor und dem mindestens einen Schalter, die die Vorrichtung aufbauen, einer reellen Funktion aus mindestens einer vorgeschlagenen Auswahl eines Funktions- und eines Identifikationsbereichs ausgerichtet ist.

2. System nach Anspruch 1,
**dadurch gekennzeichnet, dass** das Verarbeitungsprogramm die Speicherung der SensorDaten und/oder der Steuerungsdaten mit Hilfe der Speichermittel (RT3) des Funktelefons (RT) und ihre Anzeige mit Hilfe der Anzeigemittel (RT5) des Funktelefons (RT) durchführt.

3. System nach Anspruch 2,
**dadurch gekennzeichnet, dass** das Verarbeitungsprogramm zur Lenkung von sportlichen Übungen ausgelegt ist.

4. System nach einem der vorhergehenden Ansprüche,
**dadurch gekennzeichnet, dass** die Kopplungsmittel (INT) eine Vielzahl von Schnittstellenblöcken (B0, B1, B2...), einen Hauptschnittstellenblock (B0) und mindestens einen zweiten Schnittstellenblock (B1, B2...) umfassen, wobei Schnittstellenblöcke Radiofrequenz-Verbindungsmittel (MT0, MT1, MT2...) umfassen, derart, dass der Austausch von Daten zwischen dem Hauptschnittstellenblock (B0) einerseits und jedem der zweiten Schnittstellenblöcke anderseits ermöglicht wird, wobei der Hauptschnittstellenblock (B0) zum Relais gleichzeitig für die Übertragung von Daten von Sensoren, die durch die zweiten Schnittstellenblöcke (B1, B2...) in Richtung des tragbaren Funktelefons (RT) bereitgestellt werden, und für die Übertragung von Steuerungsdaten, die von dem tragbaren Funktelefon (RT) in Richtung der zweiten Schnittstellenblöcke (B1, B2...) bereitgestellt werden, dient.

5. System nach einem der vorhergehenden Ansprüche,
**dadurch gekennzeichnet, dass** die Verbindungsmittel (LC1, LC2, ...LCn) zwischen dem Kopplungsmodul (INT) und dem mindestens einen Sensor und/oder die Verbindungsmittel (LA1, LA2, ...LAn) zwischen dem Kopplungsmodul (INT) und dem mindestens einen Schalter vom Typ einer Schnurverbindung, einer Funkverbindung oder vom Typ einer optischen Verbindung sind.

6. System nach einem der vorhergehenden Ansprüche,
**dadurch gekennzeichnet, dass** die Verbindungsmittel (L) zwischen dem Kopplungsmodul (INT) und dem mobilen Funktelefon (RT) vom Typ einer Schnurverbindung gemäß den Normen "RS232" oder vom Typ einer optischen Infrarot-Verbindung oder von Typ einer Funkverbindung gemäß den Normen "Bluetooth", "Wi-FI" sind.

7. System nach einem der vorhergehenden Ansprüche,
**dadurch gekennzeichnet, dass** das Modul (COM) die Schnittstelle des Blocks (B0) und des Funktelefons (RT) aufbaut.

8. System nach einem der vorhergehenden Ansprüche,
**dadurch gekennzeichnet, dass** es die Lenkung von sportlichen Übungen ermöglicht, wobei der mindestens eine Sensor die sportliche Aktivität einer Gruppe von Anwendern misst, wobei der mindestens eine Schalter über die Mitglieder der Gruppe von Anwendern mit der Sportausrüstung verbunden ist.
